# EUROPEAN PATENT APPLICATION

(11) **EP 4 593 029 A1**
(43) Date of publication of application: **30.07.2025**
(21) Application number: 23894846.7
(22) Date of filing: 27.10.2023
(51) Int. Cl.: G16H 30/40, G16H 30/20, G06V 10/82, G06V 10/75, A61B 5/055, A61B 6/03, G06N 3/08

(54) **METHOD FOR CONVERTING MEDICAL IMAGE HAVING IMPROVED SHARPNESS, AND DEVICE THEREFOR**

(30) Priority: 24.11.2022 KR 20220158817
(71) Applicant: Polestar Healthcare Co., Ltd., Seoul 06627 (KR)
(72) Inventor: YOON, Yeo Dong, Seoul 06629 (KR); LEE, Eun Chong, Yongin-si Gyeonggi-do 16812 (KR)
(74) Representative: RGTH
(86) International application number: PCT/KR2023/016880
(87) International publication number: WO 2024/111917

(57) **Abstract**

One aspect of the present invention relates to a method for converting a generative medical image and, more specifically, to a method for converting a medical image, and a device therefor, the method allowing a learning model trained using a generative adversarial network (GAN) to learn at the level of a feature map generated during encoding of a medial image, thereby outputting a medical image having improved sharpness. According to one embodiment of the present invention, provided is the medical image conversion device, which compares, to a feature map encoded in a target image, a feature map encoded in an input image of the present invention, so as to learn to reduce the difference therebetween so that the difference in images is reduced in the feature map dimension, and thus the sharpness of an output result can be more improved.

## Description

### TECHNICAL FIELD

An aspect of the disclosure relates to a method and device for converting a medical image using artificial intelligence (Al) and, more particularly, to a method and device for converting a medical image which allows a learning model, which is trained using a generative adversarial network (GAN), to learn or be trained at the level of a feature map constructed during medical image encoding to produce a medical image having improved clarity.

### BACKGROUND ART

The information disclosed in this section is only provided for an understanding of background information of embodiments of the disclosure and should not be taken as a description of the prior art.

For the diagnosis and treatment of emergency room patients with brain hemorrhage, tumor patients, and the like, medical professionals may use non-enhanced (or non-contrast enhanced) CT images in which no contrast enhancers are used, enhanced (or contrast enhanced) CT images in which contrast enhancers are used, and the like.

Enhanced CT imaging is a type of medical imaging that uses a contrast agent to enhance tissue contrast to detect blood vessels, organs, cancerous tumors, and the like.

However, contrast agents may often cause side effects and even death due to cardiac arrest, shock death, or the like.

Because the side effects of contrast agents may not be predictable in advance, image conversion may help prevent these side effects and reduce the medical costs of contrast agents in a case in which non-enhanced CT or MR images may be converted to enhanced CT or MR images.

In addition, for individuals such as pregnant women who have MR images but no CT images due to unavailability of X-ray radiation or individuals who have CT images but are not MR imaged for reasons such as lack of time, cost, or implants, image conversion between imaging modalities may also help medical professionals to identify tissue boundaries or locate tumors without the use of contrast agents.

Furthermore, conversion between MR images, such as from a T1 image to a T2 image or from a T1 image to a diffusion image, may help medical professionals better understand lesions.

In addition, a learning model that performs machine learning may learn or be trained in the direction of extracting a feature map as input data, synthesize output data based on the feature map, comparing the synthesized output data with the target data, and reducing the difference according to a typical learning method. Because the feature map extracted in this process provides important information for generating output data, in a case in which appropriate information is not extracted from the input data, the clarity of the output result will be reduced.

The information disclosed in the Background section is technical information that the inventors possessed for, or acquired during, derivation of embodiments of the disclosure and should not be taken as known technology disclosed to the public before the filing of the embodiments of the disclosure.

### DISCLOSURE

### Technical Problem

Accordingly, an aspect of the disclosure has been made to solve the above-described problems, and an objective of the disclosure is to provide a device for converting a medical image which may further improve the clarity of an output result by learning or performing training to compare a feature map encoded in an input image with a feature map encoded in a target image and reduce the difference between the feature maps so that the difference between the images is reduced at the feature map level.

Another objective of the disclosure is to provide a method of converting a medical image which enables medical professionals to perform medical examination, diagnosis, and treatment with accurate information and patients to receive appropriate medical services.

The objectives of the disclosure are not limited to the foregoing description, and other objectives not explicitly disclosed herein will be clearly understood by a person having ordinary knowledge in the art to which the disclosure pertains from the description provided hereinafter.

### Technical Solution

To achieve at least one of the above objectives, an aspect of the disclosure provides an artificial intelligence (AI) training method for medical image conversion using a generative adversarial network (GAN).

In the training method, a third image is synthesized from a first image and a result of comparing the third image with a second image is reflected to training. The artificial intelligence training method includes:
a first operation of encoding the first image to construct a first feature map;
a second operation of encoding the second image to construct a second feature map; and
a third operation of performing training to compare the first feature map and the second feature map and to reduce a difference between the first feature map and the second feature map.

In some embodiments, the second feature map may be constructed during synthesis of the second image by encoding the second image.

In some embodiments, the first feature map may be constructed during the synthesis of the third image by encoding the first image.

In some embodiments, the difference derived in the third operation may be fed back to the process of constructing the first feature map in the first operation.

In some embodiments, the first image may be data input to an artificial intelligence learning model as training data, the third image may be an image belonging to a different domain from the first image, and the second image may form a paired data set with the first image.

Another aspect of the disclosure provides a device for converting a medical image which converts a medical image using a generative adversarial network (GAN), the device including:
a first learning model configured to extract a first image feature from a first image to construct a third image;
a second learning model configured to extract a second image feature from a second image; and
a difference extractor configured to extract a difference between the second image feature and the first image feature and feeds the difference back to the first learning model to improve clarity of the third image.

In some embodiments, the first learning model may receive the first image and constructs the third image based on the first image.

In some embodiments, the second learning model may receive the second image and generate the second image based on the second image.

In some embodiments, the first image feature may be a feature map in which the first image is encoded, and the second image feature may be a feature map in which the second image is encoded.

In some embodiments, the first image may be data input to an artificial intelligence learning model as training data, the third image may be an image belonging to a different domain from the first image, and the second image may form a paired data set with the first image.

### Advantageous Effects

According to an embodiment of the disclosure, a device for converting a medical image may further improve the clarity of an output result by learning or performing training to compare a feature map encoded in an input image with a feature map encoded in a target image and reduce the difference between the feature maps so that the difference between the images is reduced at the feature map level.

Another objective of the disclosure is to provide a method of converting a medical image which enables medical professionals to perform medical examination, diagnosis, and treatment with accurate information and patients to receive appropriate medical services.

In addition, the disclosure has a variety of effects with excellent versatility depending on the embodiment, and such effects may be clearly understood from the following description of embodiments.

### DESCRIPTION OF DRAWINGS

The following drawings accompanying the specification illustrate embodiments of the disclosure and, together with the foregoing description of the disclosure, serve to provide further understanding of the technical spirit of the disclosure, and thus, the disclosure should not be construed as being limited to the drawings, wherein:
FIG. 1 illustrates a method of converting a medical image according to an embodiment of the disclosure.
FIG. 2 illustrates an Al learning model according to an embodiment of the disclosure.
FIG. 3 illustrates an embodiment of calculating the match rate between an output image and a target image.
FIG. 4 illustrates an Al training method according to another embodiment of the disclosure.
FIG. 5 illustrates a device for converting a medical image according to another embodiment of the disclosure.

### BEST MODE

Advantages and features of the disclosure, as well as methods of realizing the same, will be more clearly understood from the following detailed description of embodiments when taken in conjunction with the accompanying drawings. However, the disclosure is not limited to specific embodiments to be described hereinafter but should be understood as including a variety of modifications, equivalents, and alternatives within the spirit and scope of the disclosure. Rather, these embodiments are provided so that the description of the disclosure will be complete and will fully convey the scope of the disclosure to a person having ordinary skill in the art in the technical field to which the disclosure pertains. In the following description of the disclosure, a detailed description of related known technology will be omitted when the description may render the subject matter of the disclosure unclear.

The terminology used herein is for the purpose of describing particular embodiments only and is not intended to be limiting. As used herein, the singular forms are intended to include the plural forms as well, unless the context clearly indicates otherwise.

Terms, such as "comprise/include" or "have," or the like, as used herein, indicate that a feature, a number, a step, an operation, a component, a part or a combination thereof described in the disclosure is present, but do not preclude the possibility of the presence or addition of one or more other features, numbers, steps, operations, components, parts or combinations thereof in advance. Although terms, such as "first", "second", or the like, may be used to describe various components, these components should not be conceived of as being limited by these terms. These terms are only used to distinguish a component from another component.

Hereinafter, embodiments according to the disclosure will be described in detail with reference to the accompanying drawings, in which identical or similar components are given the same reference numerals, and repeated descriptions thereof will be omitted.

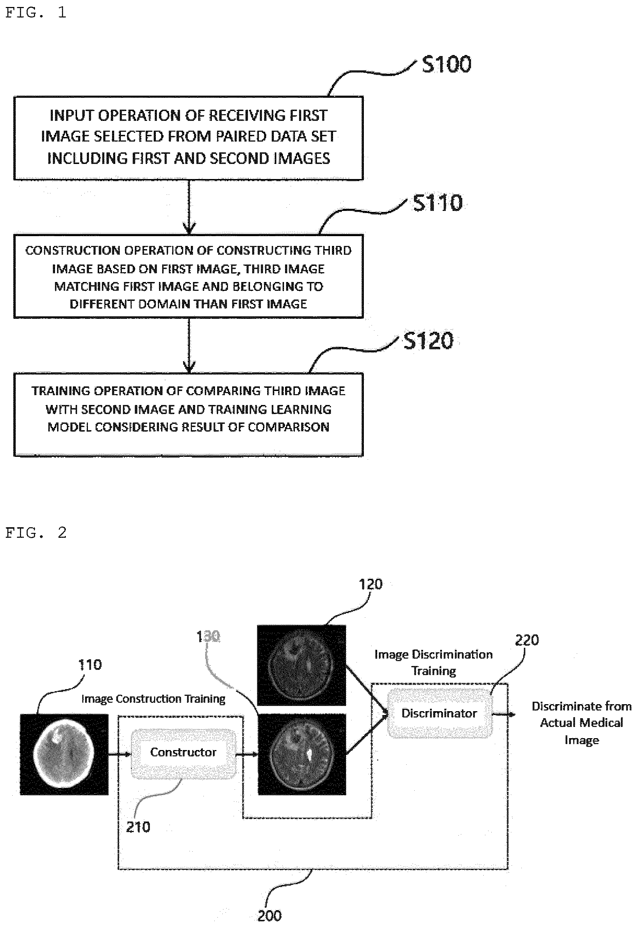

FIG. 1 illustrates a method of converting a medical image according to an embodiment of the disclosure, and FIG. 2 illustrates an artificial intelligence (AI) learning model according to an embodiment of the disclosure.

According to an embodiment of the disclosure, a method of converting a medical image using a generative adversarial network (GAN) may be provided.

The method of converting a medical image according to this embodiment may include: an input operation S100 of receiving a first image 110 selected from a paired data set including the first image 110 and a second image 120; a construction operation S110 of constructing a third image 130 based on the first image 110, the third image 130 matching the first image 110 and belonging to a different domain from the first image 110; and

a training operation S120 of comparing the third image 130 with the second image 120 and training the learning model considering a result of the comparison.

The first image 110, the second image 120, and the third image 130 may be medical images. The medical images may be magnetic resonance images, such as 2D MR, 3D MR, 2D streaming MR, 4D MR, 4D volumetric MR, and 4D cine MR images; functional MR images, such as fMR, DCE-MR, and diffusion MR images; computed tomography (CT) images, such as 2D CT, cone beam CT, 3D CT, and 4D CT images; ultrasound images, such as 2D ultrasound, 3D ultrasound, and 4D ultrasound images; positron emission tomography (PET) images; X-ray images; fluoroscopic images; radiotherapy portal images; single-photon emission computed tomography (SPECT) images; computer generated synthetic images, such as pseudo-CT images; and the like.

Furthermore, the medical images may include medical image data, such as a training image, a ground truth image, a contoured image, a dose image, and the like.

In some embodiments, the medical image may include an image acquired using an image acquisition device, a computer generated synthetic image, and the like. The image acquisition device may include, for example, an MR imaging device, a CT imaging device, a PET imaging device, an ultrasound imaging device, a fluoroscopic device, a SPECT imaging device, an integrated linear accelerator, an MR imaging device, and the like. Furthermore, the image acquisition device is not limited to the above-described examples and may include a variety of medical imaging devices within the scope of the technical idea for acquiring medical images of a patient.

A paired data set may include a set of training data including images of an object such as a particular part of a patient.

A CT imaging device and an MR imaging device may be used to acquire a CT image and an MR image, respectively, of a particular part of a patient, such as the head, in which case the CT image and the MR image may form a paired data set.

In a case in which a non-enhanced (or non-contrast enhanced) CT image and an MR image are respectively acquired for a particular part of a patient, the non-enhanced CT image and the MR image may form a paired data set. In a case in which an enhanced (or contrast enhanced) CT image and an MR image are respectively acquired, the enhanced CT image and the MR image may form a paired data set.

In a case in which T1 and T2 images are acquired from a particular part of a patient, the T1 and T2 images may form a paired data set.

In this context, the particular part of a patient may mean the particular part of a single patient. In a case in which imaged parts are the same, the imaged parts may refer to particular parts of different patients.

For example, CT and MR images acquired from a particular part of a single patient may form a paired data set, and CT and MR images acquired from the same parts of different patients may also form a paired data set.

Different domains of two images mean that the domains are different, for example, in a case in which respective patterns contained in two matching images have different contrasts or two images have different intensity distributions or different patterns.

Furthermore, images acquired using different imaging devices belong to different domains. For example, because CT and MR images are acquired using a CT imaging device and an MR imaging device, respectively, both images belong to different domains. Similarly, there are domain differences between ultrasound and CT images, between ultrasound and MR images, and between MR and PET images.

Furthermore, non-enhanced and enhanced images have different intensity distributions for the same part, and thus belong to different domains.

For MR images, T1 and T2 images are the same MR images, but have different intensity distributions and therefore belong to different domains. That is, in a case in which two images realized by reconstruction based on signals provided from an object have a difference in signals, sequences, or image realization processes on which the image realization is based, the information about the two images having the difference belongs to different domains. Furthermore, the medical image examples described above belong to different domains.

To illustrate the method of converting a medical image according to an embodiment of the disclosure, a case in which the image acquisition device has obtained a paired data set by acquiring a first image 110 and a second image 120 from the same part of a single patient will be described as an example. A case in which the first image 110 is an original CT image, the second image 120 is an original MR image, and the third image 130 is a synthetic MR image according to embodiments will be described.

The input operation S100 may include receiving, by the learning model, the first image 110 selected from the paired data set including the first image 110 and the second image 120. The first image 110 may include input data processed from an original CT image with training data.

In the paired data set including the first image 110 and the second image 120, which one of the first image 110 and the second image 120 is to be selected as the input data may be determined by the manufacturer of medical device software which performs the method of converting a medical image according to this embodiment. In this case, which image is to be selected may be selected automatically by a selection algorithm, or may be selected manually by an operator.

The manufacturer may acquire the original CT image from the CT imaging device and the original MR image from the MR imaging device to form a paired data set, and may select the original CT image as the input data from the paired data set by an algorithm or manually.

The construction operation S100 may include constructing the third image 130 based on the first image 110, in which the learning model having received the first image 110 constructs the third image 130 which matches the first image 110 and belongs to a different domain from the first image 110.

The learning model receives the original CT image as input data and constructs a synthetic MR image based on the original CT image.

The process of the learning model constructing the third image 130 from the first image 110 which belongs to a different domain from the first image 110 may be performed by the following two processes according to embodiments. However, the process of the learning model constructing the third image 130 is not limited to the following two processes.

The first process may include one or more layers which receive the first image 110 obtained from the image acquisition device and perform convolutional operations to extract high-dimensional features having a smaller size than the first image 110, in which the layers may be represented by an artificial neural network configured to extract features. Such a feature extraction artificial neural network may receive two- or three-dimensional real image data obtained from the image acquisition device and perform convolutional operations to extract image features from the original images.

Describing this process by way of example, a convolutional layer which extracts the features of an image through a filter and a pooling layer which enhances the features and reduces the size of the image may be provided to extract the features of an image by repeating the convolution and the pooling.

In some embodiments, a convolutional layer including a plurality of layers may extract features of input data given as an input from a first convolutional layer and produce a feature map as a result, a pooling layer may receive this feature map and produce a result having enhanced features and a reduced image size, and the output of the pooling layer may be input again into a second convolutional layer.

The second process includes one or more layers performing a deconvolution operation for the purpose of generating image data of different modalities from the output of the first process described above, in which these layers may be represented by an artificial neural network for generating images of different modalities. The artificial neural network which generates images of different modalities may perform a deconvolution operation on the result of the convolution operation of extracting the features by the artificial neural network in the first process to generate two- or three-dimensional image data of different modalities.

The training operation S120 may include operations of comparing the third image 130 with the second image 120 and training the learning model considering the comparison result. In a case in which the original CT image in the paired data set which includes the original CT image and the original MR image is determined to be the input data, the original MR image may be determined to be the target image. The second image 120 may correspond to ground truth. After constructing the third image 130, the learning model may be trained to reduce the difference between the constructed third image 130 and the second image 120 by comparing the constructed third image 130 with the second image 120. That is, the learning model may be trained to compare the synthetic MR image with the original MR image and continuously review whether the synthetic MR image is constructed to be similar to the original MR image, so that the synthetic MR image and the original MR image are the same. In some embodiments, an operation of calculating a loss value between the third image 130 and the second image 120 by applying a loss function to the algorithm may be included, and the parameters of the learning model may be updated based on the loss value.

In some embodiments, in the operation of calculating a loss value, the calculation of the loss value to update the learning model may be performed at each iteration. That is, the learning model may calculate the loss value between the synthetic MR image and the original MR image at each iteration during the training.

In a case in which the iteration includes a first iteration section and a second iteration section which are sequential, in some embodiments, the learning model may recognize overfitting and terminate the training in a case in which the absolute value of the change between the first loss value calculated in the first iteration section and the second loss value calculated in the second iteration section is less than a reference value.

In a case in which the learning model is sufficiently trained for the medical image conversion, the training may no longer be performed and be completed. The model having completed the training is now able to convert medical images to third images 130 with respect to a plurality of first images 110 having different data distributions, the third images 130 belonging to different domains from the first images 110.

A learning model 200 according to an embodiment of the disclosure may use a GAN model, and may include a constructor and a discriminator. The constructor may be trained to construct images, and the discriminator may be trained to discriminate images.

Referring to FIG. 2, in the learning model 200 using the GAN model, in a case in which the original CT image 110 is input as input data to a constructor 210, the constructor 210 may be trained to construct a synthetic MR image 130 by the convolutional operation described above, and a discriminator 220 may be trained to discriminate the synthetic MR image 130 from an original MR image 120, i.e., a real medical image.

In some embodiments, the learning model 200 according to this embodiment may use a cycle GAN model (not shown). In this case, the learning model 200 may include the first constructor 210, the first discriminator 220, a second constructor, and a second discriminator. In a case in which the original CT image 110 is input to the first constructor 210 as input data, the first constructor 210 may be trained to construct the synthetic MR image 130 through the convolutional operation described above, and the first discriminator 220 may be trained to discriminate a synthetic MR image from the original MR image 120, i.e., the real medical image. In a case in which the synthetic MR image 130 constructed by the first constructor 210 is then input to the second constructor, the second constructor may be trained to construct a synthetic CT image based on the synthetic MR image 130, and the second discriminator may be trained to discriminate a synthetic CT image from an original CT image.

Here, the original CT image 110 may correspond to the first image 110 in the method of converting a medical image described above, the synthetic MR image 130 may correspond to the third image 130, and the original MR image 120 may correspond to the second image 120.

In some embodiments, the training operation S120 may include calculating a match rate between the third image 130 and the second image 120 and feeding the calculated value back to the learning model. Here, calculating the match rate is a method for determining the degree of difference between two images belonging to the same domain.

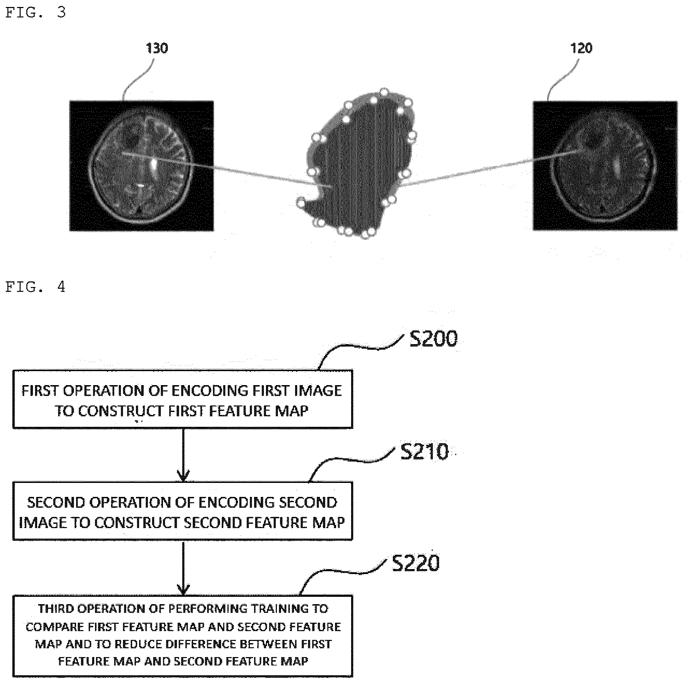

FIG. 3 illustrates an embodiment of calculating the match rate between an output image and a target image.

In some embodiments, in a case in which a lesion region is marked on each of the same parts of a synthetic MR image and an original MR image, the match rate may be calculated by experts by comparing the sizes of the lesion regions. For example, the match rate may be calculated by marking the location of a tumor or other lesion in the synthetic MR image and the original MR image, respectively, followed by conversion to a binary image.

In this case, the match rate may be calculated using an evaluation metric, such as the Hausdorff distance and the Dice similarity coefficient, or calculated quantitatively based on the difference between the centers of mass of the two lesion locations. Furthermore, in a case in which the target image and the output image are CT images, the match rate of the two images may be determined by comparing the radiation dose calculations for the lesions. In some embodiments, the determination may be performed by representing the difference between the synthetic MR image and the original MR image using numerical values such as MAE, RMSE, SSIM, or PSNR (hereinafter referred to as a performance metric).

Referring to FIG. 3, the process of calculating the match rate by aligning the lesion regions of the synthetic MR image 130, i.e., the output image, and the original MR image 120, i.e., the target image, and comparing the contours of the respective lesions using the Hausdorff distance measure is illustrated.

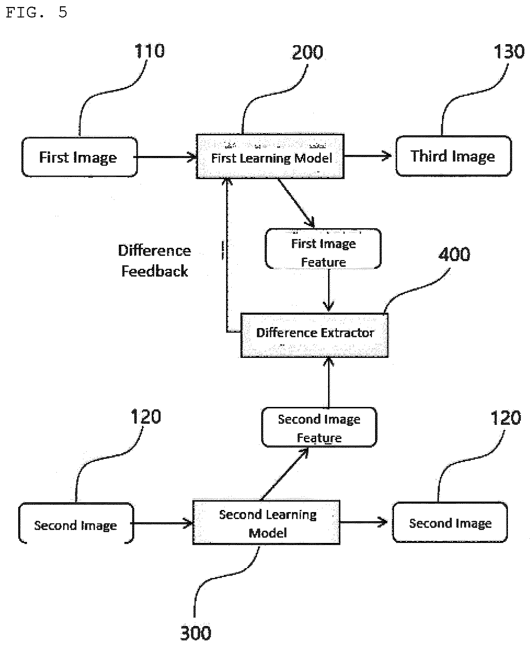

FIG. 4 illustrates an Al training method according to another embodiment of the disclosure. FIG. 5 illustrates a device for converting a medical image according to another embodiment of the disclosure.

An artificial intelligence (AI) training method according to an embodiment of the disclosure may include an Al training method for medical image conversion using a generative adversarial network (GAN).

The Al training method according to this embodiment may synthesize the third image 130 from the first image 110, and may reflect the result of comparing the third image 130 with the second image 120 to the training. For example, in a case in which the first image 110 is an original CT image, the second image 120 is an original MR image, and the third image 130 is a synthetic MR image, the Al training method according to this embodiment may receive the original CT image, construct a synthetic MR image based on the original CT image using a generative adversarial network (GAN), compare the synthetic MR image with the original MR image, and reflect the result of the comparison to the training or learning. Here, reflecting to the training includes reflecting to the training process of the Al learning model to upgrade the parameters of the learning model.

The Al training method according to this embodiment may include: a first operation S200 of encoding the first image 110 to generate a first feature map;
a second operation S210 of encoding the second image 120 to generate a second feature map; and
a third operation S220 of performing training or learning to compare the first feature map and the second feature map and to reduce a difference between the first feature map and the second feature map.

In some embodiments, the second feature map may be constructed during the synthesis of the second image 120 by encoding the second image 120.

In some embodiments, the first feature map may be constructed during the synthesis of the third image 130 by encoding the first image 110.

In some embodiments, the difference generated in the third operation S220 may be fed back to the process of constructing the first feature map in the first operation 5200.

In some embodiments, the first image 110 may be data input to the Al learning model as training data, the third image 130 may be an image belonging to a different domain from the first image 110, and the second image 120 may form a paired data set with the first image 110.

For example, in a case in which the first image 110 is an original CT image, the third image 130 is a synthetic MR image constructed based on the original CT image, and the second image 120 is an original MR image that forms a paired dataset with the original CT image,
the feature map according to this embodiment may include information in which the image features of the original CT image are compressed in a case in which the learning model compresses information about the original CT image in the process of constructing the synthetic MR image from the original CT image. For example, the feature map may be an array containing information about lines, curves, and the like. In a case in which the learning model performs a convolutional operation by receiving the original CT image, the feature map may be viewed as the result of the convolutional operation.

In this embodiment, the encoding may include extracting image feature information from the first image 110 and compressing the extracted image feature information in the process of constructing the third image 130 from the first image 110. In this embodiment, the learning model may encode the first image 110, e.g., the original CT image, to extract the first feature map, and encode the second image 120, e.g., the original MR image, to extract the second feature map. In this case, the first feature map may include the image features of the original CT image, and the second feature map may include the image features of the original MR image.

According to the disclosure, the learning model is upgraded at the feature map level by comparing the first feature map extracted in the process of constructing the third image 130 from the first image 110 and the second feature map extracted from the second image 120, which together with the first image 110 forms the paired data set, calculating the difference between the first and second feature maps, and reflecting the calculated difference to the training of the learning model. More specifically, the learning model is trained in the direction of reducing the difference between the first feature map and the second feature map. According to this embodiment, the learning model is updated by feeding the difference between the first feature map and the second feature map calculated in the third operation S220 back to the process of constructing the first feature map by the learning model in the first operation S200. The learning model then performs the training so that the first feature map is similar to the second feature map.

In this embodiment, the second feature map may be extracted during the process in which the learning model constructs the second image 120 by encoding the second image 120. That is, the second feature map may include image features of the second image 120.

The extraction of the second feature map may be performed in the process of generating the second image 120 by encoding and then decoding the second image 120, for example, in the process of compressing the original MR image and then decoding the compressed MR image to construct the original MR image. That is, the extraction of the second feature map may be performed in the process of reconstructing the original MR image. Because the second feature map extracted by this process reflects the image features of the original MR image, the second feature map may be reflected in the process of constructing the synthetic MR image from the original CT image so that the synthetic MR image accurately reflects the original MR image.

Here, in some embodiments, the training model which constructs the second feature map may be the same as or different from the above-described training model which constructs the first feature map.

The learning model constructs the synthetic MR image based on information about the compressed image features of the original CT image. Therefore, in a case in which the compressed information does not contain information suitable for conversion to MR data, the synthetic MR image constructed based on the compressed information may not properly represent the MR information, which is problematic.

In a case in which the image features compressed from the original CT image, which is the input data at the feature map level, are similar to the image features of the original MR image, which is the target image, the synthetic MR image is constructed based on the image features extracted from the original CT image. As a result, the resulting synthetic MR image may more accurately reflect the original MR image, which is the target image.

Accordingly, a synthetic MR image having improved clarity at a level comparable to the original may be produced.

According to another embodiment of the disclosure, a device for converting a medical image which converts a medical image using a generative adversarial network (GAN) may be provided.

The device for converting a medical image according to this embodiment may include: a first learning model 200 configured to extract a first image feature from a first image 110 to construct a third image 130; a second learning model 300 configured to extract a second image feature from a second image 120; and
a difference extractor 400 configured to extract a difference between the second image feature and the first image feature and feeds the difference back to the first learning model 200 to improve the clarity of the third image 130.

In some embodiments, the first learning model 200 may receive the first image 110 and construct the third image 130 based on the first image 110.

In some embodiments, the second learning model 300 may receive the second image 120 and construct the second image 120 based on the second image 120.

In some embodiments, the first image feature may include a feature map in which the first image 110 is encoded, and the second image feature may include a feature map in which the second image 120 is encoded.

As described above, in some embodiments, the first image 110 may be data input to the Al learning model as training data, the third image 130 may be an image belonging to a different domain from the first image 110, and the second image 120 may form a paired data set with the first image 110.

The device for converting a medical image according to the present embodiment may be configured to perform the method for converting a medical image of the above-described embodiment. Here, the first learning model 200 may be configured to, for example, receive an original CT image as input data, encode the original CT image to extract a feature map of the original CT image, and decode the original CT image again to produce a synthetic MR image. That is, the first learning model 200 may extract the first feature map in the process of encoding the original CT image.

The second learning model 300 may be configured to input a target image, e.g., an original MR image that forms a paired data set with the original CT image as input data, encode the original MR image to extract a feature map of the original MR image, and decode the encoded MR image again to produce the original MR image. In other words, the second learning model 300 may extract the second feature map in the process of encoding the original MR image.

In this embodiment, the difference extractor 400 may compare the first feature map extracted by the first learning model 200 and the second feature map extracted by the second learning model 300, calculate the difference between the first and second feature maps, and feed a result value of the difference calculation back to the training process of the first learning model 200. The first learning model 200 may receive the result value of the difference calculation fed back by the difference extractor 400 and update parameters of the learning model in the direction of reducing the difference between the first feature map and the second feature map.

According to the disclosure, the first learning model 200 may by be trained to reduce the difference between the first feature map and the second feature map to construct a synthetic MR image similar to the original MR image.

The above-described embodiments of the disclosure may be implemented in the form of computer programs executable on a computer using various components, and such computer programs may be stored in computer-readable media. Examples of the computer-readable media may include: magnetic media such as hard disks, floppy disks, and magnetic tapes; optical recording media such as CD-ROMs and DVDs; magneto-optical media such as floptical disks; and hardware devices such as ROMs, RAMs, and flash memories specifically configured to store program instructions and execute the program instructions.

Furthermore, the computer programs may be specifically designed and configured for the disclosure or may be known and available to a person having ordinary knowledge in the art of computer software. Examples of computer programs may include machine code produced by compilers and high-level language code executable on computers using interpreters.

In the specification (in particular Claims) of the disclosure, the use of the term "the" and similar denoting terms may correspond to both singular and plural forms. Furthermore, recitation of ranges of values herein are intended merely to refer to respective separate values falling within the respective ranges and, unless otherwise indicated herein, the respective separate values are incorporated herein as if individually recited herein.

Finally, the operations of any method described herein may be performed in any suitable order unless otherwise indicated herein or otherwise clearly contradicted by the context. However, the operations shall not be limited to the described sequence. The use of any examples or illustrative languages (e.g., "such as") provided herein, is intended merely to better illustrate the disclosure and does not pose a limitation on the scope of the disclosure unless otherwise defined by the Claims. Furthermore, a person having ordinary knowledge in the art will appreciate that various modifications, combinations, and changes are possible according to design conditions and factors within the scope of the Claims or equivalents thereof.

Therefore, the spirit of the disclosure shall not be limited to the above-described embodiments, and the entire scope of the appended claims and equivalents thereof will fall within the scope and spirit of the disclosure.

### <Description of Reference Numerals of Drawings>

110: first image
120: second image
130: third image
200: first learning model
300: second learning model
400: difference extractor

## Claims

1. An artificial intelligence training method for medical image conversion using a generative adversarial network (GAN),
wherein a third image is synthesized from a first image and a result of comparing the third image with a second image is reflected to training, the artificial intelligence training method comprising:
a first operation of encoding the first image to construct a first feature map;
a second operation of encoding the second image to construct a second feature map; and
a third operation of performing training to compare the first feature map and the second feature map and to reduce a difference between the first feature map and the second feature map.

2. The artificial intelligence training method of claim 1, wherein the second feature map is constructed during synthesis of the second image by encoding the second image.

3. The artificial intelligence training method of claim 1, wherein the first feature map is constructed during the synthesis of the third image by encoding the first image.

4. The artificial intelligence training method of claim 1, wherein the difference derived in the third operation is fed back to the process of constructing the first feature map in the first operation.

5. The artificial intelligence training method of claim 1, wherein the first image is data input to an artificial intelligence learning model as training data, the third image is an image belonging to a different domain from the first image, and the second image forms a paired data set with the first image.

6. A device for converting a medical image which converts a medical image using a generative adversarial network (GAN), the device comprising:
a first learning model configured to extract a first image feature from a first image to construct a third image;
a second learning model configured to extract a second image feature from a second image; and
a difference extractor configured to extract a difference between the second image feature and the first image feature and feeds the difference back to the first learning model to improve clarity of the third image.

7. The device of claim 6, wherein the first learning model receives the first image and constructs the third image based on the first image.

8. The device of claim 6, wherein the second learning model receives the second image and constructs the second image based on the second image.

9. The device of claim 6, wherein the first image feature is a feature map in which the first image is encoded, and the second image feature is a feature map in which the second image is encoded.

10. The device of claim 6, wherein the first image is data input to an artificial intelligence learning model as training data, the third image is an image belonging to a different domain from the first image, and the second image forms a paired data set with the first image.
